# EUROPÄISCHE PATENTSCHRIFT

(11) **EP 3 781 552 B1**
(45) Veröffentlichungstag und Bekanntmachung des Hinweises auf die Patenterteilung: **14.10.2026**
(21) Anmeldenummer: 19720073.6
(22) Anmeldetag: 15.04.2019
(51) Int. Cl.: C07D 317/72, C07C 231/02

(54) **VERFAHREN ZUR HERSTELLUNG VON ESTERN N-ACYLIERTER AMINOSÄUREN MIT SÄURELABILEN KETO-SCHUTZGRUPPENFUNKTIONEN**
METHOD FOR THE PREPARATION OF ESTERS OF N-ACYLATED AMINO ACIDS WITH ACID-LABILE KETO PROTECTION GROUP FUNCTIONS
PROCÉDÉ DE PRODUCTION D'ESTERS D'ACIDES AMINÉS N-ACYLÉS À FONCTIONS DE GROUPE DE PROTECTION KÉTO ACIDOLABILES

(30) Priorität: 17.04.2018 EP 18167708
(43) Veröffentlichungstag der Anmeldung: 24.02.2021
(73) Patentinhaber: Bayer Aktiengesellschaft, 51373 Leverkusen (DE)
(72) Erfinder: SCHNATTERER, Albert, 51373 Leverkusen (DE); DOCKNER, Michael, 50674 Köln (DE); BRÜCHNER, Peter, 47809 Krefeld (DE); HIMMLER, Thomas, 51519 Odenthal (DE)
(74) Vertreter: BIP Patents
(86) Internationale Anmeldenummer: PCT/EP2019/059641
(87) Internationale Veröffentlichungsnummer: WO 2019/201842

(56) Entgegenhaltungen:
- WO-A1-02/055481
- WO-A1-2018/024659
- DE-A1- 19 742 492
- US-A1- 2010 120 727

## Beschreibung

Die vorliegende Erfindung betrifft ein neues Verfahren zur Herstellung von Estern N-acylierter Aminosäuren aus N-acylierten Aminosäuren, die eine säurelabile Keto-Schutzgruppe enthalten, und leicht zugänglichen organischen Alkylierungsreagenzien. Die Ester N-acylierter Aminosäuren dienen als Vorstufen zur Herstellung von Pflanzenschutzmitteln mit insektizider, akarizider oder herbizider Wirkung (beispielsweise *WO 06*/*089633).*

Es ist bereits bekannt geworden, dass Aminosäuren mit Alkoholen in Gegenwart von Chlorwasserstoff zu den entsprechenden Aminosäureestern reagieren (*Houben-Weyl, XI*/*2, S. 355 ff*). Diese allgemeine Herstellmethode versagt jedoch, sobald die Aminosäuren säurelabile Schutzgruppen enthalten, die unter diesen Reaktionsbedingungen abgespalten werden.

In solchen Fällen findet die ebenfalls bereits bekannt gewordene Methode Anwendung, nach der Aminosäuren in einem polar-aprotischen Lösungsmittel mithilfe eines Alkylierungsreagenzes in Gegenwart einer Base verestert werden. So verwenden z. B. Dhavale et al. (RSC Advances 5, 81165 (2015*))* Methyliodid als Alkylierungsreagenz, Kaliumhydrogencarbonat als Base und N,N-Dimethylformamid (DMF) als Lösemittel zur Herstellung des Methylesters der (1,2-O-Isopropyliden-3-O-tosyl-5-deoxy-5-C(S)-(2(5-oxo-pyrolidin))-α-D-gluco-hexofurano)-uronsäure. Statt Kaliumhydrogencarbonat ist nach WO 02/055481 und US005770732 auch der Gebrauch von Kaliumcarbonat üblich. Meienhofer et al. (J. Org. Chem. 42, 1286 (1977*))* beschreiben die Verwendung des sehr teuren Cäsiumcarbonats als Base.

Die Beispiele aus der Literatur zeigen, dass sich die Anwendung dieser Veresterungsmethoden auf die Verwendung eines polar-aprotischen Lösungsmittels und einer schwachen Base beschränkt. Die Verwendung typischer polar-aprotischer Lösungsmittel wie beispielsweise DMF oder N,N-Dimethylacetamid (DMAC) hat aber den Nachteil, dass sich diese Lösungsmittel häufig nur unter Anwendung aufwendiger Methoden zurückgewinnen lassen, was wegen ihres relativ hohen Preises aus ökonomischen Gründen jedoch wünschenswert ist.

Es ist ebenfalls bereits bekannt geworden, die im Vergleich zu Kaliumhydrogencarbonat und Kaliumcarbonat stärkere Base Kaliumhydroxid einzusetzen. Hierbei wird nach Creighton et al. (J. Am. Chem. Soc. 121, 6786 (1999*))* jedoch nicht nur der Sauerstoff der Carboxylgruppe, sondern auch der der hier durch eine tert-Butyloxycarbonyl-Gruppe (Boc) geschützte Stickstoff der Aminosäure alkyliert.

Weiterhin kann die in Lehrbüchern der Organischen Chemie (z. B. J. March, Advanced organic chemistry, 3. Auflage, John Wiley & Sons 1985, S. 354, ISBN 0-471-85472-7*)* beschriebene Methode zur Veresterung von Carbonsäuren mit Diazoalkan auf Aminosäuren übertragen werden, wie unter anderem aus US20100120727 bekannt geworden ist. Doch aus sicherheitstechnischen und ökonomischen Gründen werden Diazoalkane im Wesentlichen nur im Laborbereich eingesetzt und kommen für eine technische Anwendung im großen Maßstab kaum in Frage.

Es bestand demnach weiterhin Bedarf, ein breiter anwendbares, sicheres und ökonomisches technisches Verfahren zur Herstellung von Estern N-acylierter Aminosäuren, die säurelabile Keto-Schutzgruppen tragen, zur Verfügung zu stellen.

Überraschenderweise wurde nun gefunden, dass die Veresterung von N-acylierten Aminosäuren, die eine säurelabile Keto-Schutzgruppe enthalten, unter alkalischen Bedingungen ohne Verwendung eines polar-aprotischen Lösungsmittels gelingt, indem die in situ hergestellte N-acylierte Aminosäure mit säurelabiler Keto-Schutzgruppe mithilfe eines Alkylhalogenids oder eines Mono- oder Dialkyl-esters der Schwefelsäure verestert wird.

Gegenstand der vorliegenden Erfindung ist somit ein neues Verfahren zur Herstellung von Verbindungen der allgemeinen Formel (I), 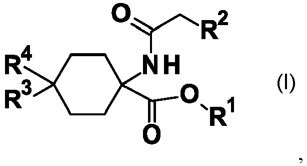 in der
- R¹: für geradkettiges oder verzweigtes C₁-C₆-Alkyl oder Benzyl steht,
- R²: für geradkettiges oder verzweigtes C₁-C₆-Alkyl oder gegebenenfalls durch Methyl, Ethyl, Fluor, Chlor, Methoxy oder Ethoxy substituiertes Phenyl steht,
- R³ und R⁴: unabhängig voneinander für einen Rest OR⁵ oder SR⁵ stehen oder gemeinsam für einen Rest -O(CHR⁶)ₙO- oder gemeinsam für einen Rest =NR⁷ stehen,
wobei
- R⁵: für geradkettiges oder verzweigtes C₁ - C₆-Alkyl steht,
- R⁶: für Wasserstoff, Methyl, Ethyl oder Phenyl steht,
- n: für 2 oder 3 steht,
- R⁷: für geradkettiges oder verzweigtes C₁-C₆-Alkyl, Phenyl, Benzyl oder 4-Methoxybenzyl steht,
dadurch gekennzeichnet, dass man in einem ersten Schritt (1) Aminosäuresalze der allgemeinen Formel (II) 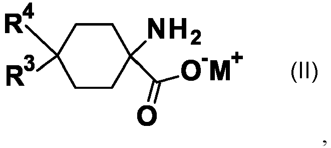 in der
- M: für Natrium, Kalium oder eine Gruppe NR⁸₄ steht,
wobei
- R⁸: für Wasserstoff oder geradkettiges oder verzweigtes C₁-C₆-Alkyl steht, und
- R³ und R⁴: die oben angegebene Bedeutung haben,
mit Carbonsäurehalogeniden der allgemeinen Formel (III) 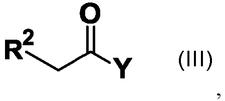 in der
- Y: für Fluor, Chlor oder Brom steht und
- R²: die oben angegebene Bedeutung hat,
zu N-acylierten Aminosäuresalzen der allgemeinen Formel (IV) 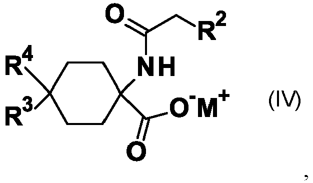 in der
M, R², R³ und R⁴ die oben angegebenen Bedeutungen haben,
in Gegenwart einer Base und Wasser und eines Lösungsmittels oder Lösungsmittelgemisches, welches nicht polar-aprotisch ist, umsetzt und im Anschluss daran in einem zweiten Schritt (2) des erfindungsgemäßen Verfahrens die N-acylierten Aminosäuren der allgemeinen Formel (IV) mit einem Alkylierungsreagenz der allgemeinen Formel (V) oder (VI) in Gegenwart einer Base und eines Lösungsmittels oder Lösungsmittelgemisches, welches nicht polar-aprotisch ist, zu den Verbindungen der allgemeinen Formel (I) umsetzt, wobei folgende Lösungsmittel als nicht polar-aprotisch gelten:
Toluol, o-Xylol, m-Xylol, p-Xylol, Mesitylen, Chlorbenzol, 1,2-Dichlorbenzol, Anisol, Cyclohexan, Methyl-cyclohexan, Pentan, Heptan, Isooctan oder Gemische dieser Lösungsmittel.

Als Alkylierungsreagenzien finden Alkylhalogenide der allgemeinen Formel (V) oder Schwefelsäuredi- oder -monoester oder Salze der Schwefelsäuremonoester der allgemeinen Formel (VI) Verwendung 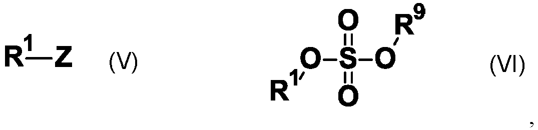 in denen
- R¹: die oben angegebene Bedeutung hat,
- Z: für Chlor, Brom oder Iod steht
und
- R⁹: für Wasserstoff, Natrium, Kalium oder den Rest R¹ steht.

Die Verbindungen der Formel (II) und (III) sind entweder kommerziell erhältlich oder nach bekannten Verfahren herstellbar.

Die Verbindungen der Formeln (V) und (VI) sind kommerziell erhältlich.

Das erfindungsgemäße Verfahren wird durch das Schema 1 veranschaulicht.

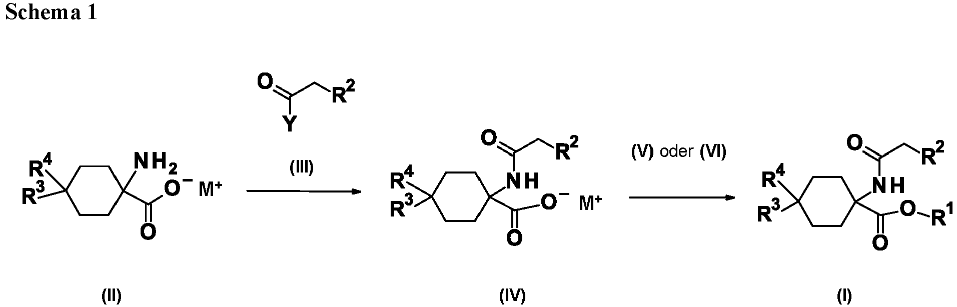

Bevorzugt ist das Verfahren zur Herstellung von Verbindungen der allgemeinen Formel (I), wobei
- R¹: für Methyl, Ethyl, n-Propyl, n-Butyl oder Benzyl steht,
- R²: für gegebenfalls durch Methyl, Ethyl, Chlor, Methoxy oder Ethoxy substituiertes Phenyl steht,
- R³ und R⁴: unabhängig voneinander für einen Rest OR⁵ oder gemeinsam für einen Rest -O(CHR⁶)ₙO- oder gemeinsam für einen Rest =NR⁷ stehen,
- R⁵: für geradkettiges C₁ - C₆-Alkyl steht,
- R⁶: für Wasserstoff, Methyl, Ethyl oder Phenyl steht,
- n: für 2 oder 3 steht,
- R⁷: für geradkettiges oder verzweigtes C₁-C₆-Alkyl, Phenyl, Benzyl oder 4-Methoxybenzyl steht,
- M: für Natrium oder Kalium steht,
- Y: für Fluor oder Chlor steht,
- Z: für Chlor, Brom oder Iod steht,
- R⁹: für Wasserstoff, Natrium, Kalium oder den Rest R¹ steht.

Besonders bevorzugt ist das Verfahren zur Herstellung von Verbindungen der allgemeinen Formel (I),
wobei
- R¹: für Methyl, Ethyl, n-Propyl oder n-Butyl steht,
- R²: für gegebenenfalls durch Methyl, Ethyl oder Chlor substituiertes Phenyl steht,
- R³ und R⁴: für einen Rest OR⁵ oder gemeinsam für einen Rest -O(CH₂)₂O- stehen,
- R⁵: für Methyl, Ethyl, n-Propyl oder n-Butyl steht,
- M: für Natrium oder Kalium steht,
- Y: für Chlor steht,
- Z: für Brom oder Iod steht,
- R⁹: für Wasserstoff, Natrium, Kalium oder den Rest R¹ steht.

Hervorgehoben ist das Verfahren zur Herstellung der Verbindung der Formel (I-1), 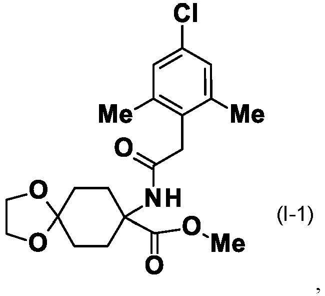 dadurch gekennzeichnet, dass man in einem ersten Schritt (1) das Aminosäuresalz der allgemeinen Formel (II-1) 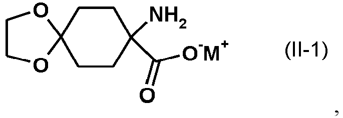 in der
- M: für Natrium oder Kalium steht,
mit dem Carbonsäurehalogenid der Formel (III-1) 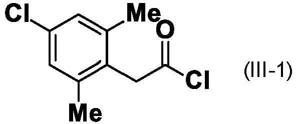 zu N-acylierten Aminosäuresalzen der allgemeinen Formel (IV-1) 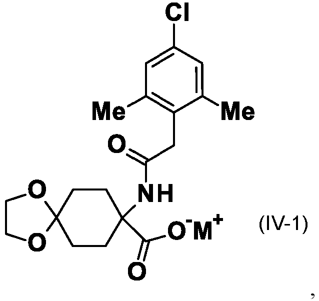 in der
- M: für Natrium oder Kalium steht,
in Gegenwart einer Base und Wasser und eines Lösungsmittels oder Lösungsmittelgemisches, welches nicht polar-aprotisch ist, umsetzt und im Anschluss daran in einem zweiten Schritt (2) des erfindungsgemäßen Verfahrend die N-acylierten Aminosäuren der allgemeinen Formel (IV-1) mit Dimethylsulfat (Verbindung der Formel (VI-1), in welcher R¹ und R⁹ für Methyl stehen) in Gegenwart einer Base und eines Lösungsmittels oder Lösungsmittelgemisches, welches nicht polar-aprotisch ist, zu der Verbindung der Formel (I-1) umsetzt, wobei folgende Lösungsmittel als nicht polar-aprotisch gelten:
Toluol, o-Xylol, m-Xylol, p-Xylol, Mesitylen, Chlorbenzol, 1,2-Dichlorbenzol, Anisol, Cyclohexan, Methyl-cyclohexan, Pentan, Heptan, Isooctan oder Gemische dieser Lösungsmittel.

Beispielhaft genannt sind Verbindungen der allgemeinen Formel (I-a) 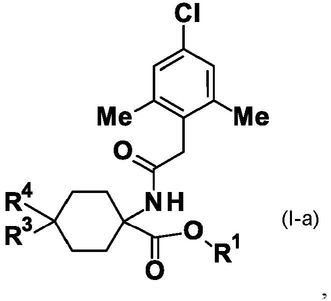 in der
- R¹: für Methyl, Ethyl, n-Propyl, n-Butyl oder Benzyl steht,
und
- R³ und R⁴: für einen Rest OR⁵ oder gemeinsam für einen Rest -O(CH₂)₂O- stehen, wobei
- R⁵: für Methyl, Ethyl, n-Propyl oder n-Butyl steht,

wobei wenn R¹ für Methyl steht, dann steht R⁵ nicht für Methyl (siehe WO 2018/024659),
wobei wenn R¹ für Methyl steht, dann stehen R³ und R⁴ nicht gemeinsam für einen Rest -O(CH₂)₂O-(siehe WO 2018/024659).

In DE 19742492 wird die folgende Verbindung offenbart:

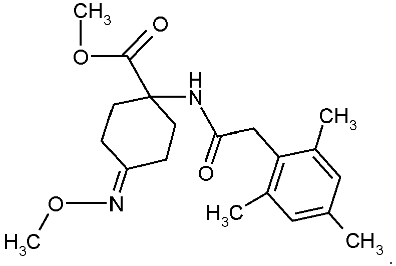

Bevorzugt sind Verbindungen der allgemeinen Formel (I-a), in der
- R¹: für Ethyl, n-Propyl oder n-Butyl steht
und
- R³ und R⁴: gemeinsam für einen Rest -O(CH₂)₂O- stehen.

Beispielhaft genannt sind Verbindungen der allgemeinen Formel (IV-1) 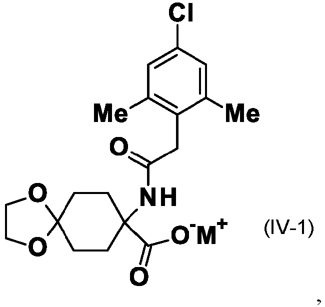 in der
- M: für Natrium oder Kalium steht.

Im Folgenden wird das erfindungsgemäße Verfahren näher erläutert:
Im Einzelnen wird im erfindungsgemäßen Verfahren so vorgegangen, dass man im ersten Schritt (1) zunächst Aminosäuresalze der allgemeinen Formel (II) in Wasser oder einer wässrigen Lösung einer Base löst oder diese Aminosäuresalze der allgemeinen Formel (11) erzeugt, indem man die entsprechenden freien Aminosäuren oder Salze der Aminosäuren mit Säuren wie beispielsweise Hydrochloride, Sulfate oder Hydrosulfate, in einer wässrigen Lösung einer Base löst.

Als Basen kommen beispielsweise in Betracht Lithiumcarbonat, Natriumcarbonat, Natriumhydrogencarbonat, Kaliumcarbonat, Kaliumhydrogencarbonat, Lithiumhydroxid, Natriumhydroxid oder Kaliumhydroxid oder Gemische dieser Basen. Bevorzugt verwendet man Natriumcarbonat, Natriumhydrogencarbonat, Kaliumcarbonat, Kaliumhydrogencarbonat, Natriumhydroxid oder Kaliumhydroxid oder Gemische dieser Basen.

Die Menge an Base wird so gewählt, dass ein pH-Wert von 8 bis 14 eingestellt wird. Bevorzugt stellt man einen pH-Wert 10,5 bis 12,5 ein.

Gegebenenfalls kann es notwendig sein, den pH-Wert durch nachträgliche Zugabe einer anorganischen Säure auf den gewünschten Bereich einzustellen. Als anorganische Säuren werden Salzsäure oder Schwefelsäure, bevorzugt Salzsäure verwendet
Anschließend wird in diesem ersten Schritt (1) des erfindungsgemäßen Verfahrens die wässrige Lösung der Aminosäuresalze der allgemeinen Formel (II) mit einem Carbonsäurehalogenid der allgemeinen Formel (III) zu einem N-acylierten Aminosäuresalz der allgemeinen Formel (IV) umgesetzt.

Die Menge an Carbonsäurehalogenid der allgemeinen Formel (III) beträgt dabei 0,9 bis 1,5 Moläquivalente, bezogen auf das Aminosäuresalz der allgemeinen Formel (II). Bevorzugt setzt man 1,0 bis 1,25 Moläquivalente ein.

Das Carbonsäurehalogenid der allgemeinen Formel (III) wird entweder ohne Verwendung eines Lösungsmittels in flüssiger Form oder als Lösung in einem unter den Reaktionsbedingungen inerten Lösungsmittel zugegeben. Als Lösungsmittel kommen beispielsweise in Betracht Toluol, o-Xylol, m-Xylol, p-Xylol, Mesitylen, Chlorbenzol, 1,2-Dichlorbenzol, Anisol, Cyclohexan, Methyl-cyclohexan, Pentan, Heptan, Isooctan oder Gemische dieser Lösungsmittel. Bevorzugt verwendet man Toluol, o-Xylol, m-Xylol, p-Xylol, Mesitylen, Chlorbenzol, Anisol, Methyl-cyclohexan, Heptan, Isooctan oder Gemische dieser Lösungsmittel. Besonders bevorzugt verwendet man Toluol.

Falls zur Aufrechterhaltung des gewünschten pH-Wertes erforderlich, wird gleichzeitig zu der Dosierung des Carbonsäurehalogenids der allgemeinen Formel (III) weitere wässrige Basenlösung dosiert. Dabei wird entweder die zum Carbonsäurehalogenid äquimolare Menge an Base parallel dosiert, oder die Reaktion wird unter pH-Kontrolle durchgeführt und die Dosierung der Base entsprechend angepasst.

Der erste Schritt (1) des erfindungsgemäßen Verfahrens wird beispielsweise bei einer Temperatur zwischen 0 und 100°C, bevorzugt zwischen 10 und 70°C durchgeführt.

Die N-acylierten Aminosäuresalze der allgemeinen Formel (IV) können isoliert werden oder man setzt die wässrigen Lösungen der N-acylierten Aminosäuresalze der allgemeinen Formel (IV) ohne Aufarbeitung in den zweiten Schritt des erfindungsgemäßen Verfahrens ein. Bevorzugt setzt man die wässrigen Lösungen ohne weitere Aufarbeitung ein.

Sollen die N-acylierten Aminosäuresalze der allgemeinen Formel (IV) isoliert werden, kann dies beispielsweise durch Einengen der wässrigen Lösungen im Vakuum erfolgen. Eine Methode des Verfahrens zur Isolierung der N-acylierten Aminosäuresalze der allgemeinen Formel (IV) besteht darin, die Kationenkonzentration (Natrium oder Kalium) in der Lösung durch Zugabe von beispielsweise Natriumhydroxid, Natriumcarbonat, Natriumhydrogencarbonat, Natriumchlorid, Natriumsulfat, Kaliumhydroxid, Kaliumcarbonat, Kaliumhydrogencarbonat, Kaliumchlorid oder Kaliumsulfat zu erhöhen. Dadurch kommt es entweder zu der Ausbildung einer zweiten wässrigen Phase, die das N-acylierte Aminosäuresalz enthält, oder das N-acylierte Aminosäuresalz fällt aus und kann abfiltriert werden.

Im zweiten Schritt (2) des erfindungsgemäßen Verfahrens werden die N-acylierten Aminosäuresalze der allgemeinen Formel (IV) mit einem Alkylierungsmittel der allgemeinen Formel (V) oder (VI) zu den Aminosäureestern der allgemeinen Formel (I) umgesetzt. Bevorzugt verwendet man Dimethylsulfat als Alkylierungsmittel.

Das Alkylierungsmittel wird in Mengen von 1 bis 5 Moläquivalenten, bezogen auf das N-acylierte Aminosäuresalz der allgemeinen Formel (IV), eingesetzt. Bevorzugt verwendet man 1,5 bis 2,5 Moläquivalente.

Während der Dosierung des Alkylierungsmittels der allgemeinen Formel (V) oder (VI) wird durch gleichzeitige Zugabe einer Base der pH-Wert der Reaktionsmischung zwischen 8 bis 14, bevorzugt zwischen 8 bis 12,5 gehalten.

Als Basen kommen beispielsweise in Betracht Lithiumcarbonat, Natriumcarbonat, Natriumhydrogencarbonat, Kaliumcarbonat, Kaliumhydrogencarbonat, Lithiumhydroxid, Natriumhydroxid oder Kaliumhydroxid oder Gemische dieser Basen. Bevorzugt verwendet man Natriumcarbonat, Natriumhydrogencarbonat, Kaliumcarbonat, Kaliumhydrogencarbonat, Natriumhydroxid oder Kaliumhydroxid oder Gemische dieser Basen.

Die Reaktionstemperatur im zweiten Schritt (2) des erfindungsgemäßen Verfahrens kann in weiten Grenzen variiert werden. Man wird die Reaktionstemperatur einerseits möglichst hoch wählen, um eine schnelle und vollständige Umsetzung zu erreichen. Andererseits wird man die Reaktionstemperatur so niedrig wählen, dass eine alkalische Verseifung des gebildeten N-acylierten Aminosäureesters der allgemeinen Formel (I) möglichst nicht stattfindet. Dementsprechend hängt die Reaktionstemperatur auch vom gewählten pH-Wert im zweiten Schritt (2) des erfindungsgemäßen Verfahrens ab. Sie liegt typischerweise zwischen 0 und 120°C, bevorzugt zwischen 15 und 90°C.

Der zweite Schritt (2) des erfindungsgemäßen Verfahrens kann sowohl ohne als auch in Gegenwart eines Phasentransferkatalysators durchgeführt werden. Bevorzugt führt man die Reaktion unter Zusatz eines Phasentransferkatalysators durch.

Die Menge an Phasentransferkatalysator liegt typischerweise zwischen 0,01 und 0,2 Moläquivalenten, bevorzugt zwischen 0,08 und 0,12 Moläquivalenten.

Als typische Phasentransferkatalysatoren seien beispielhaft genannt: Tri-n-butyl-n-tetradecylphosphoniumchlorid, Tetraphenylphosphoniumbromid, Tetrabutylammoniumbromid, Tetrabutylammoniumhydrogensulfat, Tetraoctylammoniumchlorid oder Tetradecylammoniumchlorid oder Gemische solcher Tetra-alkyl-ammoniumsalze wie beispielsweise Aliquat336.

Bevorzugt verwendet man Tri-n-butyl-n-tetradecylphosphoniumchlorid, Tetraoctylammoniumchlorid wie beispielsweise Aliquat 336, Tetradecylammoniumchlorid oder Gemische dieser Tetra-alkyl-ammoniumsalze. Besonders bevorzugt verwendet man Aliquat 336.

Der zweite Schritt (2) des erfindungsgemäßen Verfahrens kann außer bei Normaldruck auch sowohl bei vermindertem als auch erhöhtem Druck durchgeführt werden.

Die Wahl der Aufarbeitungsmethode wird durch die Eigenschaften des hergestellten Aminosäureesters bestimmt.

Die vorliegende Erfindung wird durch die folgenden Beispiele näher erläutert ohne durch sie eingeschränkt zu sein.

### Beispiele

### Beispiel 1: Natrium-8-[2-(4-chlor-2,6-dimethylphenyl)acetamido]-1,4-dioxaspiro[4.5]decan-8-carboxylat

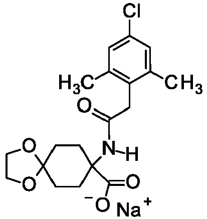

In einem 600 mL-Reaktionsgefäß mit Überkopfrührer, pH-Elektrode und Dosiereinrichtung wird eine Lösung von 47,3 g Natrium-8-amino-1,4-dioxaspiro[4.5]decan-8-carboxylat einer Reinheit von 70,8% (entspricht 150 mMol; der Rest ist im Wesentlichen Natriumcarbonat und Natriumhydroxid) in 108 mL Wasser vorgelegt. Der pH-Wert der leicht trüben Lösung liegt bei 12,9. Man kühlt auf 10°C und stellt durch Zugabe von 10%iger Salzsäure den pH-Wert auf 11,8. Anschließend dosiert man innerhalb einer Stunde eine Lösung von 36,5 g [168 mMol] (4-Chlor-2,6-dimethylphenyl)acetylchlorid in 23 mL Toluol zu. Gleichzeitig werden 25,1 g 32%ige Natronlauge [201 mMol NaOH] so zudosiert, dass der pH-Wert konstant bei 11,8 bleibt. Nach erfolgter Dosierung rührt man noch für eine Stunde bei 10°C, lässt auf Raumtemperatur kommen und trennt die Phasen. Man erhält 220 g trübe gelbe Lösung, die ohne weitere Aufarbeitung in den nächsten Schritt eingesetzt werden kann. Eine HPLC-Analyse (sauer) zeigt einen Anteil von 75,4% 8-[2-(4-Chlor-2,6-dimethylphenyl)acetamido]-1,4-dioxaspiro[4.5]decan-8-carbonsäure (neben 24,0% 4-Chlor-2,6-dimethylphenyl-essigsäure und 0,1% Toluol).

### Beispiel 2: Natrium-8-[2-(4-chlor-2,6-dimethylphenyl)acetamido]-1,4-dioxaspiro[4.5]decan-8-carboxylat

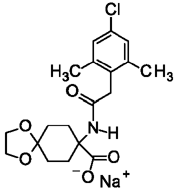

In einem 100 mL-Reaktionsgefäß mit Überkopfrührer, pH-Elektrode und Dosiereinrichtung wird eine Lösung von 18,92 g Natrium-8-amino-1,4-dioxaspiro[4.5]decan-8-carboxylat einer Reinheit von 70,8% (entspricht 60 mMol; der Rest ist im Wesentlichen Natriumcarbonat und Natriumhydroxid) in 43 mL Wasser vorgelegt. Man kühlt auf 10°C und stellt durch Zugabe von 10%iger Salzsäure den pH-Wert auf 11,8. Anschließend dosiert man innerhalb einer Stunde eine Lösung von 14,33 g [66 mMol] (4-Chlor-2,6-dimethylphenyl)acetylchlorid in 7,5 mL Toluol zu. Gleichzeitig werden 23,6 g 32%ige Natronlauge [188 mMol NaOH] so zudosiert, dass der pH-Wert konstant bei 11,8 bleibt. Nach erfolgter Dosierung rührt man noch für eine Stunde bei 10°C, lässt auf Raumtemperatur kommen und trennt die Phasen. Eine Drittel der wässrigen Phase wird bei Raumtemperatur mit 9,1 g 32%iger Natronlauge versetzt, wodurch ein Feststoff ausfällt. Dieser Feststoff wird abfiltriert und getrocknet Es resultieren 1,8 g gelblicher Feststoff, der nach quantitativer ¹H-NMR-Analyse zu 69,2% aus der Titelverbindung besteht.

HPLC-Analyse (sauer): 82,6% 8-[2-(4-Chlor-2,6-dimethylphenyl)acetamido]-1,4-dioxaspiro[4.5]decan-8-carbonsäure (neben 14,1% 4-Chlor-2,6-dimethylphenyl-essigsäure).
Ionenchromatographie: 7,26% Natrium (theoretischer Wert. 5,7%)
¹H-NMR (600 MHz, D₂O): δ = 1,6 -1,7 (m; 2H). 1,73 - 1,8 (m; 2H), 1,9 - 2 (m;2H), 2,05 -2,13 (m; 2H), 2,3 (s; 6H), 3,7 (s; 2H), 4,03 (s; 4H), 7,12 (s; 2H) ppm.

Aus dem Filtrat fällt weiterer Feststoff aus. Nach Trocknen erhält man 3,4 g Feststoff, der nach quantitativer ¹H-NMR-Analyse zu 67,3% aus der Titelverbindung besteht.

Beide Anteile Feststoff summieren sich zu einer Ausbeute von 44% der Theorie (hochgerechnet für den gesamten Ansatz).

Ein zweites Drittel der wässrigen Phase wird bei 50°C mit 9,1 g 32%iger Natronlauge versetzt, wodurch ein Feststoff ausfällt. Man rührt 15 Minuten bei 50°C, lässt auf Raumtemperatur abkühlen und rührt weitere 30 Minuten. Der Feststoff wird abfiltriert und getrocknet Es resultieren 9,1 g gelblicher Feststoff, der nach quantitativer ¹H-NMR-Analyse zu 67,8% aus der Titelverbindung besteht, womit sich eine Ausbeute von 76,5% der Theorie ergibt (hochgerechnet für den gesamten Ansatz).

### Beispiel 3: Kalium-8-[2-(4-chlor-2,6-dimethylphenyl)acetamido]-1,4-dioxaspiro[4.5]decan-8-carboxylat

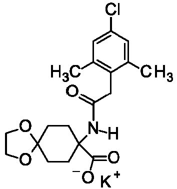

In einem 100 mL-Reaktionsgefäß mit Überkopfrührer, pH-Elektrode und Dosiereinrichtung wird eine Lösung von 18,84 g Kalium-8-amino-1,4-dioxaspiro[4.5]decan-8-carboxylat einer Reinheit von 76,2% (entspricht 60 mMol; der Rest ist im Wesentlichen Kaliumcarbonat und Kaliumhydroxid) in 43 mL Wasser vorgelegt. Man kühlt auf 10°C und stellt durch Zugabe von 10%iger Salzsäure den pH-Wert auf 11,8. Anschließend dosiert man innerhalb einer Stunde eine Lösung von 14,33 g [66 mMol] (4-Chlor-2,6-dimethylphenyl)acetylchlorid in 6,5 mL Toluol zu. Gleichzeitig werden 22,9 g 45%ige Kalilauge [184 mMol KOH] so zudosiert, dass der pH-Wert konstant bei 11,8 bleibt. Nach erfolgter Dosierung rührt man noch für eine Stunde bei 10°C, lässt auf Raumtemperatur kommen und trennt die Phasen. Eine Hälfte der wässrigen Phase wird mit 13,7 g 45 %iger Kalilauge versetzt, wodurch sich zwei Phasen bilden. Man trennt die Phasen und engt die untere Phase (24,7 g) im Vakuum ein. Es resultieren 15,7 g gelblicher Feststoff, der nach quantitativer ¹H-NMR-Analyse zu 67,7% aus der Titelverbindung besteht, womit sich eine Ausbeute von 84,4% der Theorie ergibt (hochgerechnet für den gesamten Ansatz). HPLC-Analyse (sauer): 77,9% 8-[2-(4-Chlor-2,6-dimethylphenyl)acetamido]-1,4-dioxaspiro[4.5]decan-8-carbonsäure (neben 12,4% 4-Chlor-2,6-dimethylphenyl-essigsäure und 9,3% Toluol). Ionenchromatographie: 11,6% Kalium (theoretischer Wert. 9,3%).

¹H-NMR (600 MHz, D₂O): δ = 1,5 -1,6 (m; 2H). 1,65 - 1,7 (m; 2H), 1,8 - 1,9 (m;2H), 1,95 -2 (m; 2H), 2,22 (s; 6H), 3,67 (s; 2H), 4 (s; 4H), 7,09 (s; 2H) ppm.

### Beispiel 4: Methyl-8-[2-(4-chlor-2,6-dimethylphenyl)acetamido]-1,4-dioxaspiro[4.5]decan-8-carboxylat

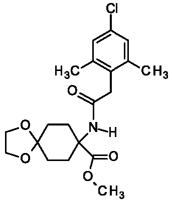

In einem 1000 mL-Reaktionsgefäß mit Überkopfrührer, pH-Elektrode, Strömungsbrecher und Dosiereinrichtung werden 310,0 g [0,486 mol] einer 16,6%igen Lösung von Natriumcarbonat in Wasser, 15,4 g Wasser und 110,4 g [0,371 mol] Natrium-8-amino-1,4-dioxaspiro[4.5]decan-8-carboxylat mit einer Reinheit von 75,0% (der Rest ist im Wesentlichen Natriumcarbonat und Natriumhydroxid) bei Raumtemperatur vorgelegt. Der pH-Wert der Suspension liegt bei 13,9. Bei 20°C stellt man durch Zugabe von 37,8 g einer 18,8%igen Salzsäure den pH-Wert auf 11,8. Anschließend dosiert man in dreieinhalb Stunden eine Lösung von 88,8 g [0,409 mol] (4-Chlor-2,6-dimethylphenyl)acetylchlorid in 67,6 g Toluol zu. Nach erfolgter Dosierung rührt man noch eine Stunde bei 20°C, gibt 166,1 g Toluol zu und erwärmt die Reaktionsmischung auf 80°C. Bei 80°C werden 3,1 g [0,007 mol, Reinheit 99%] Methyl- tri-n-octylammoniumchlorid (Aliquat 336) zugegeben und anschließend in zwei Stunden 136,1 g [1,074 mol, Reinheit 99,5%] Dimethylsulfat zudosiert. Vor dem Abkühlen auf 20°C wird noch eine halbe Stunde bei 80°C nachgerührt. Schließlich wird das während der Dimethylsulfatdosierung ausgefallene Produkt abfiltriert und der Nutschkuchen zweimal mit jeweils 458 g Wasser und zweimal mit jeweils 176 g Toluol gewaschen. Nach dem Trocknen erhält man 115,6 g [0,285 mol] Methyl-8-[2-(4-chlor-2,6-dimethylphenyl)acetamido]-1,4-dioxaspiro[4.5]decan-8-carboxylat in einer Reinheit von 97,6% (HPLC, externer Standard). Dies entspricht einer Ausbeute von 77%.

### Beispiel 5: Methyl-8-[2-(4-chlor-2,6-dimethylphenyl)acetamido]-1,4-dioxaspiro[4.5]decan-8-carboxylat

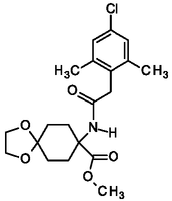

In einem 1000 mL-Reaktionsgefäß mit Überkopfrührer, pH-Elektrode und Dosiereinrichtung wird eine Lösung von 159,5 g [0,600 mol] Natrium-8-amino-1,4-dioxaspiro[4.5]decan-8-carboxylat einer Reinheit von 80,4% (der Rest ist im Wesentlichen Natriumcarbonat und Natriumhydroxid) in 441,8 g Wasser vorgelegt. Der pH-Wert der leicht trüben Lösung liegt bei 13,3. Man kühlt auf 10°C und stellt durch Zugabe von 8,2 g einer 31%igen Salzsäure den pH-Wert auf 11,8. Anschließend dosiert man in zweieinhalb Stunden eine Lösung von 130,0 g [0,599 mol] (4-Chlor-2,6-dimethylphenyl)acetylchlorid in 113,4 Toluol zu. Gleichzeitig werden 86,9 g [0,695 mol NaOH] 32%ige Natronlauge so zudosiert, dass der pH-Wert konstant bei 11,8 bleibt. Nach erfolgter Dosierung rührt man noch für eine Stunde bei 10°C und hält dabei durch weitere Zugabe von 32%iger Natronlauge den pH bei 11,8. Die Reaktionsmischung wird auf 20°C erwärmt. Bei 20°C gibt man 24,6 g [0,060 mol, Reinheit 99%] Methyl- tri-n-octylammoniumchlorid zu und dosiert in eineinhalb Stunden 153,0 g Dimethylsulfat [1,201 mol, Reinheit 99,0%] zu. Parallel zur Dimethylsulfatdosierung werden 23,0 g [0,184 mol NaOH] 32%ige Natronlauge so zudosiert, dass der pH-Wert konstant bei 11,8 bleibt. Die Reaktionsmischung wird eineinhalb Stunden bei 20°C nachgerührt und dabei der pH durch weitere Zugabe von 32%iger Natronlauge bei 11,8 gehalten. In der Nachrührphase wird die Reaktionsmischung mit 146,1 g Toluol versetzt, um den sich bildenden Feststoff besser dispergieren zu können. Am Ende der Nachrührzeit wird der Feststoff abfiltriert und nacheinander mit 300 g Wasser und dreimal mit jeweils 150 g Toluol gewaschen. Nach dem Trocknen des Feststoffs erhält man 214,3 g [0,482 mol] des gewünschten Produkts in einer Reinheit von 89,1% (HPLC, externer Standard). Dies entspricht einer Ausbeute von 80%.

### Beispiel 6: 11-(4-Chlor-2,6-dimethylphenyl)-12-hydroxy-1,4-dioxa-9-azadispiro[4.2.48.25]tetra dec-11-en-10-on (nicht erfindungsgemäß)

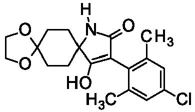

Man legt 4,9 g [12,37 mMol] Methyl-8-[2-(4-chlor-2,6-dimethylphenyl)acetamido]-1,4-dioxaspiro[4.5]-decan-8-carboxylat in 20 ml wasserfreiem N,N-Dimethylformamid vor und gibt dann 5,57 g [30,9 mMol] einer 30%igen Lösung von NaOMe in Methanol zu. Das Reaktionsgemisch wird für drei Stunden auf 80°C erwärmt und das Methanol abdestilliert. Anschließend rührt man noch 16 Stunden bei 110°C. Das Reaktionsgemisch wird bei Raumtemperatur in eine Mischung aus 100 mL Wasser und 25 mL Eisessig eingerührt. Der ausgefallene Feststoff wird abgesaugt, zweimal mit Wasser gewaschen und getrocknet. Man erhält 4,4 g hell-beigen Feststoff einer Reinheit nach HPLC-Analyse von 97,3%. Dies entspricht einer Ausbeute von 95% der Theorie.

## Patentansprüche

1. Verfahren zur Herstellung von Verbindungen der Formel (I) 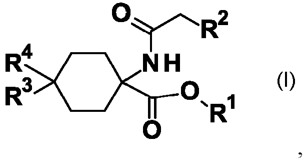 in welcher
R¹ für geradkettiges oder verzweigtes C₁-C₆-Alkyl oder Benzyl steht,
R² für geradkettiges oder verzweigtes C₁-C₆-Alkyl oder gegebenenfalls durch Methyl, Ethyl, Fluor, Chlor, Methoxy oder Ethoxy substituiertes Phenyl steht,
R³ und R⁴ unabhängig voneinander für einen Rest OR⁵ oder SR⁵ stehen oder gemeinsam für einen Rest -O(CHR⁶)ₙO- oder gemeinsam für einen Rest =NR⁷ stehen, wobei
R⁵ für geradkettiges oder verzweigtes C₁-C₆-Alkyl steht,
R⁶ für Wasserstoff, Methyl, Ethyl oder Phenyl steht,
n für 2 oder 3 steht,
R⁷ für geradkettiges oder verzweigtes C₁-C₆-Alkyl, Phenyl, Benzyl oder 4-Methoxybenzyl steht,
**dadurch gekennzeichnet, dass** man Verbindungen der Formel (II) 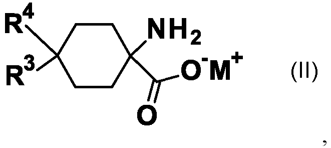 in welcher
M für Natrium, Kalium oder eine Gruppe NR⁸₄ steht, wobei
R⁸ für Wasserstoff oder für geradkettiges oder verzweigtes C₁-C₆-Alkyl steht
und
R³ und R⁴ die oben angegebene Bedeutung haben,
mit Verbindungen der Formel (III) 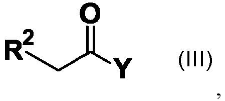 in welcher
Y für Fluor, Chlor oder Brom steht und
R² die oben angegebene Bedeutung hat,
zu Verbindungen der Formel (IV) 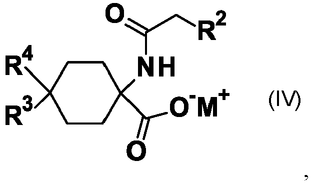 in welcher
M, R², R³ und R⁴ die oben angegebenen Bedeutungen haben,
in Gegenwart einer Base und Wasser und eines Lösungsmittels oder Lösungsmittelgemisches, welches nicht polar-aprotisch ist, umsetzt und im Anschluss daran die Verbindungen der Formel (IV) mit einem Alkylierungsreagenz der Formel (V) oder (VI) 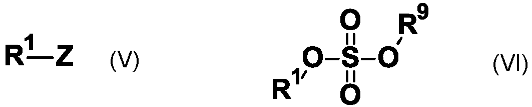
in welchen
R¹ die oben angegebene Bedeutung hat,
Z für Chlor, Brom oder Iod steht und
R⁹ für Wasserstoff, Natrium, Kalium oder den Rest R¹ steht,
in Gegenwart einer Base und eines Lösungsmittels oder Lösungsmittelgemisches, welches nicht polar-aprotisch ist, umsetzt, wobei folgende Lösungsmittel als nicht polar-aprotisch gelten:
Toluol, o-Xylol, m-Xylol, p-Xylol, Mesitylen, Chlorbenzol, 1,2-Dichlorbenzol, Anisol, Cyclohexan, Methyl-cyclohexan, Pentan, Heptan, Isooctan oder Gemische dieser Lösungsmittel.

2. Verfahren gemäß Anspruch 1, wobei
R¹ für Methyl, Ethyl, n-Propyl, n-Butyl oder Benzyl steht,
R² für gegebenenfalls durch Methyl, Ethyl, Chlor, Methoxy oder Ethoxy substituiertes Phenyl steht,
R³ und R⁴ unabhängig voneinander für einen Rest OR⁵ oder gemeinsam für einen Rest -O(CHR⁶)ₙO- oder gemeinsam für einen Rest =NR⁷ stehen,
R⁵ für geradkettiges C₁-C₆-Alkyl steht,
R⁶ für Wasserstoff, Methyl, Ethyl oder Phenyl steht,
n für 2 oder 3 steht,
R⁷ für geradkettiges oder verzweigtes C₁-C₆-Alkyl, Phenyl, Benzyl oder 4-Methoxybenzyl steht,
M für Natrium oder Kalium steht,
Y für Fluor oder Chlor steht,
Z für Chlor, Brom oder Iod steht,
R⁹ für Wasserstoff, Natrium, Kalium oder den Rest R¹ steht.

3. Verfahren gemäß Anspruch 1, wobei
R¹ für Methyl, Ethyl, n-Propyl oder n-Butyl steht,
R² für gegebenenfalls durch Methyl, Ethyl oder Chlor substituiertes Phenyl steht,
R³ und R⁴ für einen Rest OR⁵ oder gemeinsam für einen Rest -O(CH₂)₂O- stehen,
R⁵ für Methyl, Ethyl, n-Propyl oder n-Butyl steht,
M für Natrium oder Kalium steht,
Y für Chlor steht,
Z für Brom oder Iod steht,
R⁹ für Wasserstoff, Natrium, Kalium oder den Rest R¹ steht.

4. Verfahren zur Herstellung der Verbindung der Formel (I-1) gemäß Anspruch 1 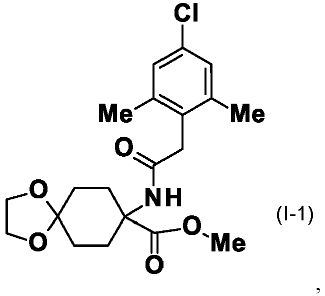 **dadurch gekennzeichnet, dass** man die Verbindung der Formel (II-1) 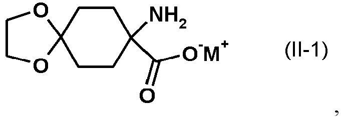 in welcher
M für Natrium oder Kalium steht,
mit der Verbindung der Formel (III-1) 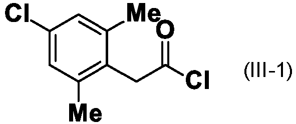 zu Verbindungen der Formel (IV-1) 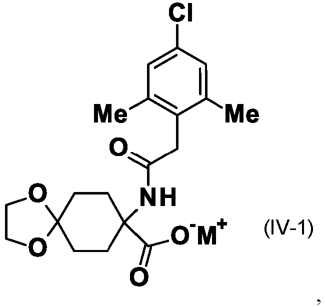 in welcher
M für Natrium oder Kalium steht,
in Gegenwart einer Base und Wasser und eines Lösungsmittels oder Lösungsmittelgemisches, welches nicht polar-aprotisch ist, umsetzt und im Anschluss daran die Verbindungen der Formel (IV-1) mit Dimethylsulfat der Formel (VI-1) in Gegenwart einer Base und eines Lösungsmittels oder Lösungsmittelgemisches, welches nicht polar-aprotisch ist, umsetzt wobei folgende Lösungsmittel als nicht polar-aprotisch gelten:
Toluol, o-Xylol, m-Xylol, p-Xylol, Mesitylen, Chlorbenzol, 1,2-Dichlorbenzol, Anisol, Cyclohexan, Methyl-cyclohexan, Pentan, Heptan, Isooctan oder Gemische dieser Lösungsmittel.

5. Verfahren zur Herstellung der Verbindung der Formel (I-1) gemäß Anspruch 4 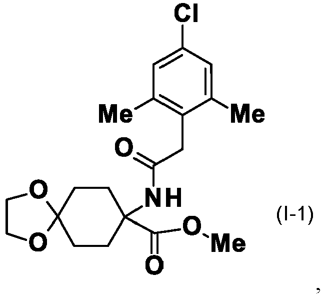 **dadurch gekennzeichnet, dass** man die Verbindung der Formel (II-1) 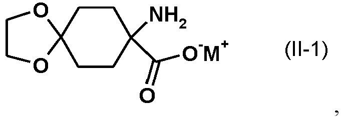 in welcher
M für Natrium oder Kalium steht,
mit der Verbindung der Formel (III-1) 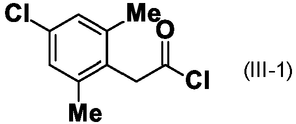 zu Verbindungen der Formel (IV-1) 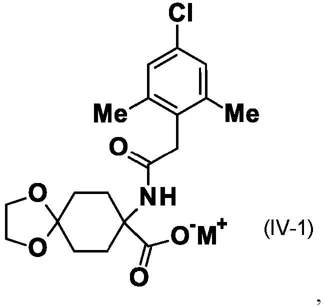 in welcher
M für Natrium oder Kalium steht,
in Gegenwart einer Base und Wasser und Toluol umsetzt und im Anschluss daran die Verbindungen der Formel (IV-1) mit Dimethylsulfat der Formel (VI-1) in Gegenwart einer Base und Toluol umsetzt.

## Claims

1. A method for the preparation of compounds of formula (I) 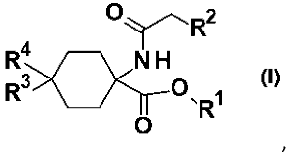 in which
R¹ represents a straight-chain or branched C₁-C₆-alkyl or benzyl group,
R² represents a straight-chain or branched C₁-C₆-alkyl group or a phenyl group optionally substituted by methyl, ethyl, fluoro, chloro, methoxy or ethoxy,
R³ and R⁴ independently of one another represent a moiety OR⁵ or SR⁵, or together represent a moiety -O(CHR⁶)ₙO- or together represent a moiety =NR⁷,wherein
R⁵ represents a straight-chain or branched C₁-C₆-alkyl group,
R⁶ represents hydrogen, methyl, ethyl or phenyl,
n represents 2 or 3,
R⁷ represents a straight-chain or branched C₁-C₆-alkyl group, phenyl, benzyl or 4-methoxybenzyl,
**characterized in that** reacting compounds of formula (II) 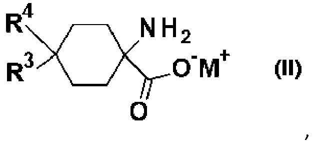 in which
M represents sodium, potassium or a group NR⁸₄,
wherein
R⁸ represents hydrogen or a straight-chain or branched C₁-C₆-alkyl group
and
R³ and R⁴ have the meanings given above,
with compounds of formula (III) 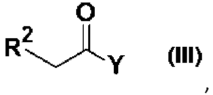 in which
Y represents fluoro, chloro or bromo, and
R² has the meaning given above,
to compounds of formula (IV) 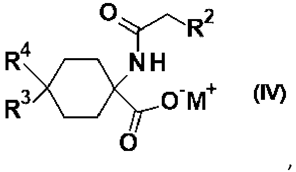 in which
M, R², R³ and R⁴ have the meanings given above,
in the presence of a base and water and a solvent or solvent mixture which is not polar-aprotic, and subsequently reacting the compounds of formula (IV) with an alkylating reagent of formula (V) or (VI) 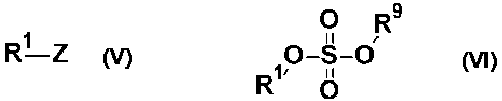
in which
R¹ has the meaning given above,
Z represents chloro, bromo or iodo
and
R⁹ represents hydrogen, sodium, potassium or the moiety R¹,
in the presence of a base and a solvent or solvent mixture which is not polar-aprotic, wherein the following solvents are considered to be not polar-aprotic:
toluene, o-xylene, m-xylene, p-xylene, mesitylene, chlorobenzene, 1,2-dichlorobenzene, anisole, cyclohexane, methylcyclohexane, pentane, heptane, isooctane or mixtures of these solvents.

2. A method according to claim 1, wherein
R¹ represents methyl, ethyl, n-propyl, n-butyl or benzyl,
R² represents a phenyl group optionally substituted by methyl, ethyl, chloro, methoxy or ethoxy,
R³ and R⁴ independently of one another represent a moiety OR⁵, or together represent a moiety -O(CHR⁶)ₙO-, or together represent a moiety =NR⁷,
R⁵ represents a straight-chain C₁-C₆-alkyl group,
R⁶ represents hydrogen, methyl, ethyl or phenyl,
n represents 2 or 3,
R⁷ represents a straight-chain or branched C₁-C₆-alkyl group, phenyl, benzyl or 4-methoxybenzyl,
M represents sodium or potassium,
Y represents fluoro or chloro,
Z represents chloro, bromo or iodo,
R⁹ represents hydrogen, sodium, potassium or the moiety R¹.

3. A method according to claim 1, wherein
R¹ represents methyl, ethyl, n-propyl or n-butyl,
R² represents a phenyl group optionally substituted by methyl, ethyl or chloro,
R³ and R⁴ represent a moiety OR⁵ or, together, a moiety -O(CH₂)₂O-,
R⁵ represents methyl, ethyl, n-propyl or n-butyl,
M represents sodium or potassium,
Y represents chloro,
Z represents bromo or iodo,
R⁹ represents hydrogen, sodium, potassium or the moiety R¹.

4. A method for the preparation of the compound of formula (I-1) as claimed in claim 1 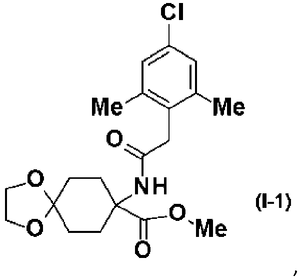 **characterized in that** reacting the compound of formula (II-1) 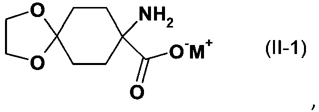 in which
M represents sodium or potassium, 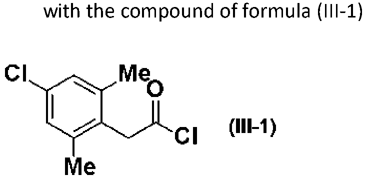
to compounds of formula (IV-1) 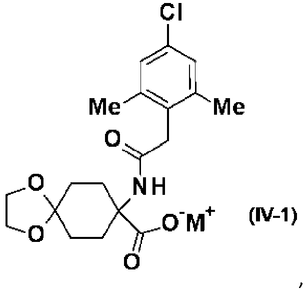 in which
M represents sodium or potassium,
in the presence of a base and water and a solvent or solvent mixture which is not polar-aprotic, and subsequently reacting the compounds of formula (IV-1) with dimethyl sulphate of formula (VI-1) in the presence of a base and a solvent or solvent mixture which is not polar-aprotic, wherein the following solvents are considered to be not polar-aprotic:
toluene, o-xylene, m-xylene, p-xylene, mesitylene, chlorobenzene, 1,2-dichlorobenzene, anisole, cyclohexane, methylcyclohexane, pentane, heptane, isooctane or mixtures of these solvents.

5. A method for the preparation of the compound of formula (I-1) as claimed in claim 4 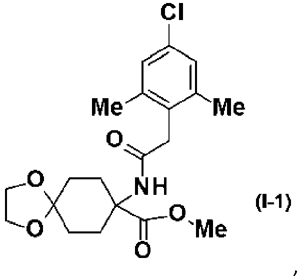 **characterized in that** reacting the compound of formula (II-1) 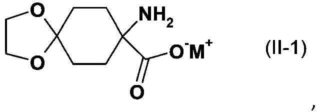 in which
M represents sodium or potassium,
with the compound of formula (III-1) 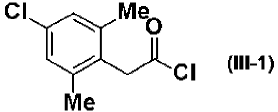 to compounds of formula (IV-1) 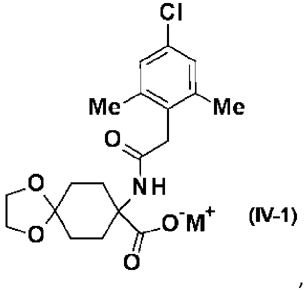 in which
M represents sodium or potassium,
in the presence of a base and water and toluene, and subsequently reacting the compounds of formula (IV-1) with dimethyl sulphate of formula (VI-1) in the presence of a base and toluene.

## Revendications

1. Procédé de préparation de composés de formule (I) 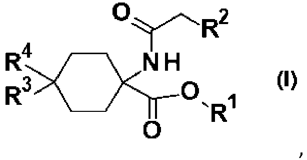 dans laquelle
R¹ représente un groupe alkyle en C₁-C₆ à chaîne linéaire ou ramifiée, ou un groupe benzyle,
R² représente un groupe alkyle en C₁-C₆ à chaîne linéaire ou ramifiée, ou un groupe phényle éventuellement substitué par un groupe méthyle, éthyle, fluoro, chloro, méthoxy ou éthoxy,
R³ et R⁴ représentent, indépendamment l'un de l'autre, un radical OR⁵ ou SR⁵, ou représentent ensemble un radical -O(CHR⁶)ₙO-, ou encore ensemble un radical =NR⁷,
où
R⁵ représente un groupe alkyle en C₁-C₆ à chaîne linéaire ou ramifiée,
R⁶ représente un atome d'hydrogène, un groupe méthyle, éthyle ou phényle,
n représente 2 ou 3,
R⁷ représente un groupe alkyle en C₁-C₆ à chaîne linéaire ou ramifiée, un groupe phényle, benzyle ou 4-méthoxybenzyle,
**caractérisé en ce que** l'on fait réagir des composés de formule (II) 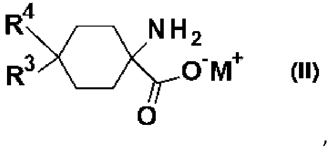 dans laquelle
M représente le sodium, le potassium ou un groupe NR⁸₄, où
R⁸ représente un atome d'hydrogène ou un groupe alkyle en C₁-C₆ à chaîne linéaire ou ramifiée et
R³ et R⁴ ont la signification indiquée ci-dessus,
avec des composés de formule (III) 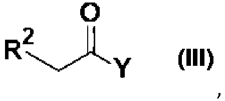 dans laquelle
Y représente le fluoro, le chloro ou le bromo, et
R² a la signification indiquée ci-dessus,
pour obtenir des composés de formule (IV) 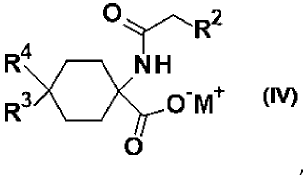 dans laquelle
M, R², R³ et R⁴ ont les significations indiquées ci-dessus,
en faisant réagir en présence d'une base, d'eau et d'un solvant ou d'un mélange de solvants qui n'est pas polaire-aprotique, puis en faisant réagir les composés de formule (IV) avec un réactif d'alkylation de formule (V) ou (VI) 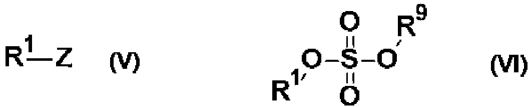
dans lesquelles
R¹ a la signification indiquée ci-dessus,
Z représente le chloro, le bromo ou l'iodo
et
R⁹ représente l'hydrogène, le sodium, le potassium ou le radical R¹,
en présence d'une base et d'un solvant ou d'un mélange de solvants qui n'est pas polaire-aprotique, les solvants suivants étant considérés comme non polaires-aprotiques :
le toluène, l'o-xylène, le m-xylène, le p-xylène, le mésitylène, le chlorobenzène, le 1,2-dichlorobenzène, l'anisole, le cyclohexane, le méthylcyclohexane, le pentane, l'heptane, l'isooctane ou des mélanges de ces solvants.

2. Procédé selon la revendication 1, où
R¹ représente un groupe méthyle, éthyle, n-propyle, n-butyle ou benzyle,
R² représente un groupe phényle éventuellement substitué par un groupe méthyle, éthyle, chloro, méthoxy ou éthoxy,
R³ et R⁴ représentent, indépendamment l'un de l'autre, un radical OR⁵ ou, conjointement, un radical -O(CHR⁶)ₙO- ou, conjointement, un radical =NR⁷,
R⁵ représente un groupe alkyle en C₁-C₆ à chaîne linéaire,
R⁶ représente un atome d'hydrogène, un groupe méthyle, éthyle ou phényle,
n représente 2 ou 3,
R⁷ représente un groupe alkyle en C₁-C₆ à chaîne linéaire ou ramifiée, un groupe phényle, benzyle ou 4-méthoxybenzyle,
M représente le sodium ou le potassium,
Y représente le fluoro ou le chloro,
Z représente le chloro, le bromo ou l'iodo,
R⁹ représente l'hydrogène, le sodium, le potassium ou le radical R¹.

3. Procédé selon la revendication 1, où
R¹ représente un groupe méthyle, éthyle, n-propyle ou n-butyle,
R² représente un groupe phényle éventuellement substitué par un groupe méthyle, éthyle ou chloro,
R³ et R⁴ représentent un radical OR⁵ ou, ensemble, un radical -O(CH₂)₂O-,
R⁵ représente un groupe méthyle, éthyle, n-propyle ou n-butyle,
M représente le sodium ou le potassium,
Y représente le chloro,
Z représente le bromo ou l'iodo,
R⁹ représente l'hydrogène, le sodium, le potassium ou le radical R¹.

4. Procédé de préparation du composé de formule (I-1) selon la revendication 1 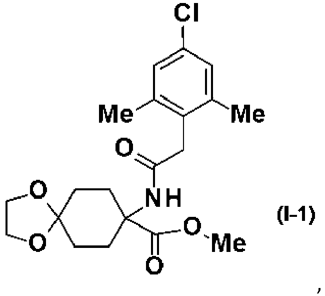 **caractérisé en ce que** l'on fait réagir le composé de formule (II-1) 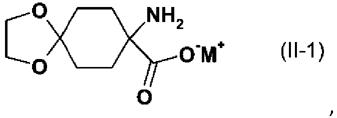 dans laquelle
M représente le sodium ou le potassium,
avec le composé de formule (III-1) 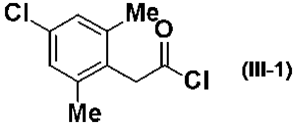 pour obtenir des composés de formule (IV-1) 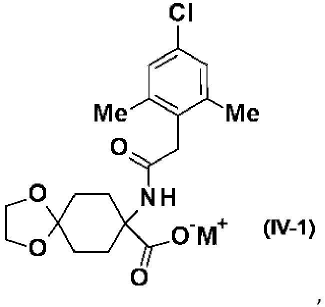 dans laquelle
M représente le sodium ou le potassium,
en faisant réagir en présence d'une base, d'eau et d'un solvant ou d'un mélange de solvants qui n'est pas polaire-aprotique, puis en faisant réagir les composés de formule (IV-1) avec du sulfate de diméthyle de formule (VI-1) en présence d'une base et d'un solvant ou d'un mélange de solvants qui n'est pas polaire-aprotique, les solvants suivants étant considérés comme non polaires-aprotiques :
le toluène, l'o-xylène, le m-xylène, le p-xylène, le mésitylène, le chlorobenzène, le 1,2-dichlorobenzène, l'anisole, le cyclohexane, le méthylcyclohexane, le pentane, l'heptane, l'isooctane ou des mélanges de ces solvants.

5. Procédé de préparation du composé de formule (I-1) selon la revendication 4 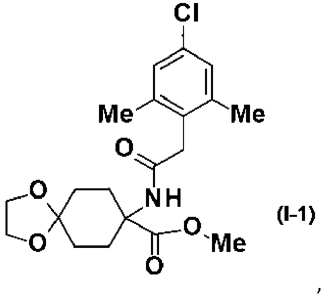 **caractérisé en ce que** l'on fait réagir le composé de formule (II-1) 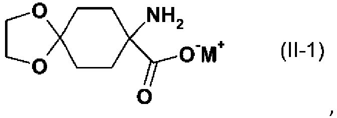 dans laquelle
M représente le sodium ou le potassium,
avec le composé de formule (III-1) 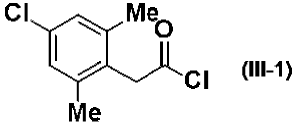 pour obtenir des composés de formule (IV-1) 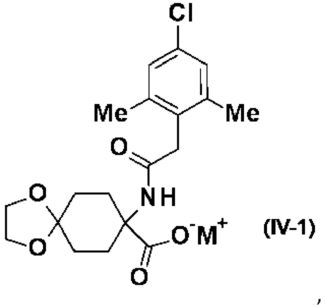 dans laquelle
M représente le sodium ou le potassium,
en faisant réagir en présence d'une base, d'eau et de toluène, puis en faisant réagir les composés de formule (IV-1) avec du sulfate de diméthyle de formule (VI-1) en présence d'une base et de toluène.
